# EUROPÄISCHE PATENTSCHRIFT

(11) **EP 1 308 154 B1**
(45) Veröffentlichungstag und Bekanntmachung des Hinweises auf die Patenterteilung: **18.05.2005**
(21) Anmeldenummer: 02090358.9
(22) Anmeldetag: 21.10.2002
(51) Int. Cl.: A61K 7/48

(54) **Kosmetikum mit erhöhter Feuchthaltewirkung**
Cosmetic composition with moisturizing effect
Composition cosmétique à effect hydratant

(30) Priorität: 31.10.2001 DE 10154979
(43) Veröffentlichungstag der Anmeldung: 07.05.2003
(73) Patentinhaber: Coty B.V., 2031 CC Haarlem (NL)
(72) Erfinder: Golz-Berner, Karin, 98000 Monaco (MC); Zastrow, Leonhard, 98000 Monaco (MC)
(74) Vertreter: Walter, Wolf-Jürgen

(56) Entgegenhaltungen:
- EP-A- 1 142 904
- WO-A-01/64046
- DATABASE WPI Section Ch, Week 198934 Derwent Publications Ltd., London, GB; Class D21, AN 1989-246392 XP002263736 & JP 01 180809 A (NOEVIER KK), 18. Juli 1989 (1989-07-18)

## Beschreibung

Die Erfindung betrifft ein Kosmetikum mit einer speziell erhöhten Feuchthaltewirkung auf natürlicher Basis.

Kosmetische Produkte enthalten in den meisten Fällen Feuchthaltemittel (Moisturizer), die einerseits eine stabile Konsistenz des Produktes gewährleisten, andererseits aber einen positiven Einfluß auf den Zustand und das Aussehen der Haut ausüben sollen. Da sich mit zunehmenden Alter der Zustand des Kollagens in der Haut verändert und die Wasserbindungsfähigkeit abnimmt, erhöht sich in den oberen Hautschichten, insbesondere im Stratum corneum die Trockenheit, was mit einer Faltenbildung einhergeht sowie weiteren Verschlechterungen wie Schuppenbildung, pergamentartiges Aussehen usw. Auch äußere Einflüsse, wie Gebrauch von Lösungsmitteln, extremes Waschen und Entfetten der Haut sowie Witterungseinflüsse können zu dieser Verschlechterung des Hautzustandes beitragen.

Natürlich in der Haut vorkommende Faktoren, zu denen Harnstoff und Aminosäuren wie Glycin und Arginin gehören, werden durch synthetische Feuchthaltemittel wie Polyole, Glycerin, Sorbit, ethoxylierte Phenole, Zuckerkomplexe, Wollwachsderivate und hydrolysierte Proteine unterstützt, wobei deren Stabilität nicht immer befriedigend ist und auch mögliche Hautirritationen nicht auszuschließen sind.

JP 1180809 offenbart ein Kosmetikum mit mildem Scheuereffekt das 1-10 Gew.% pulverförmige Teilchen von Spongiidae Schwämmen enthält.

Aus der EP 1142904 ist bekannt, daß extrahierte Schwammproteine keinen Schutz für Haut und Haar darstellen und nur eine geringe feuchtigkeitshaltende Wirkung haben. Erst deren Hydrolysate in flüssiger Form sollen auch feuchtigkeitshaltende Eigenschaften neben anderen Eigenschaften haben.

Der Erfindung liegt die Aufgabe zugrunde, ein Feuchhaltemittel für ein Kosmetikum bereitzustellen, das eine stabile Zubereitung gewährleistet, im wesentlichen unveränderten natürlichen Ursprungs ist und eine deutlich erhöhte Feuchtigkeitslieferung für die Haut mit sich bringt sowie als Puder einen Mattierungseffekt zeigt.

Das erfindungsgemäße Kosmetikum enthält 0,01 bis 5 Gew-%, bezogen auf das Gesamtgewicht des Kosmetikums, elastische Fasergerüstteilchen der Hornschwammarten Euspongia officinalis, Spongia usitatissima, Hippospongia equina und Gemische davon, wobei die Teilchengrößen im Bereich von 10 bis 200 µm liegen, sowie 95 bis 99,99 Gew-% kosmetische Trägerstoffe, Hilfsstoffe, Wirkstoffe und Gemische davon.

Die Fasergerüstteilchen bestehen aus gewaschenen und entkalkten Schwämmen, die ohne weitere Vorbehandlung nach Zerkleinerung auf die erforderliche Teilchengröße in dem Kosmetikum eingesetzt werden.

Besonders vorteilhaft sind Teilchengrößen von 30 bis 80 µm. Die Einhaltung der Teilchengrößen ist notwendig, um eine Feuchthaltewirkung in merklichem Umfang realisieren zu können. Bei zu kleinen Teilchengrößen unter 10 µm wird keine ausreichende Wirkung erkennbar.

Durch eine Kombination von feinteiliger Perlensubstanz, die allgemeinen aus Aragonit und/oder Calcit und organischen Bindemitteln (Conchagene) besteht, kann eine weitere Verbesserung des Feuchthaltevermögens erreicht werden, insbesondere kann die Feuchthaltewirkung noch weiter verlängert werden. Die Teilchengröße liegt dabei etwa im gleichen Bereich wie die der Schwammteilchen, kann aber auch darunter liegen bis 0,1 µm. Der Anteil der Perlensubstanz kann im Bereich von 0,01 bis 5 Gew-% liegen.

Eine weitere vorteilhafte Kombination besteht darin, daß die Schwammteilchen mit Hyaluronsäure, Hyaluronsäurederivaten wie z.B das Na-, K-, Zn-salz oder Propylenglycolhyaluronat oder mit einem Hyaluronat-Wirkstoff-Wasser-Komplex (gemäß DE-A-19923829) in Kontakt gebracht wird. Insbesondere bei dem genannten Komplex können Wirkstoffe wie z.B. Vitamine, Vitaminderivate; Enzym-Vitamingemische; Pflanzenextrakte wie Rosmarinextrakt, der wäßrige Extrakt der Ananasfrucht mit einem Bromelinanteil von 0,1-1 Gew-% bezogen auf das Extraktgewicht, der alkoholische Extrakt von Äpfeln in den Komplex eingebunden werden und stehen für eine deutlich verlängerte Zeit gegenüber der normalen Zumischung in die kosmetische Emulsion oder das Gel zur Verfügung.
Außerdem ergibt sich eine Wechselwirkung zu dem o.g. Hyaluronat-Wirkstoff-Wasser-Komplex und dem in den Schwammteilchen gespeicherten Wasser dahingehend, daß auch der Entzug des Wassers aus den Schwammteilchen verzögert erfolgt und somit für die Haut eine "dosierte" Zuführung von Wasser und Wirkstoff erfolgen kann.

Der Anteil an Hyaluronsäure, einem Derivat davon oder dem o.g. Komplex kann im Bereich von 0,01 bis 2,5 Gew-% liegen, bezogen auf das Gesamtgewicht des Kosmetikums.

Das gegebenenfalls als weiteren Wirkstoff einzusetzende Enzym-Vitamingemisch ist vorzugsweise ein durch Ultraschallbehandlung hergestelltes Aufschlußprodukt einer Hefe, wobei das Aufschlußprodukt SOD, Protease, Vitamin B₂, Vitamin B₆, Vitamin B₁₂, Vitamin D₂ und Vitamin E enthält. Vorzugsweise enthält es wenigstens 150 U/ml SOD, Protease und die Vitamine B und D, wobei das Verhältnis SOD:Protease als internationale Einheiten wenigstens im Bereich von 3:1 bis 8:1 liegt.

Besonders vorteilhaft für die Herstellung des Enzym-/Vitamingemisches ist ein Aufschlußverfahren mittels Ultraschall, das in DE 4241154C1 (US-A-5629185) beschrieben ist und bei dem in einer Ultraschall-Durchflußzelle eine Zelldispersion oder Suspension durch einen Beschallungsraum geführt wird, bei dem die Sonotrode zur Hälfte bis Zweidrittel ihrer Länge in die Durchflußzelle hineinragt und in das zu beschallende Medium eintaucht. Dabei hat die Sonotrode einen Winkel von 80,5 bis 88,5°, und das Verhältnis der Eintauchlänge der Sonotrode in mm zum Beschallungsvolumen in ml wird auf einen Wert von 1:1,1 bis 1:20 eingestellt. Der Feststoffanteil in dem zu beschallenden Medium liegt im Bereich von 1:0,02 bis 1:2,2 (in Gew.-%). Als Zelldispersion können Hefen, wie Bäckerhefe, Brauereihefe, Weinhefe sowie besonders behandelte Hefen, wie z.B. SOD-angereicherte Hefen, eingesetzt werden. Eine vorteilhaft einzusetzende Zelldispersion enthält z. B. Saccharomyces cerevisiae.

Das Enzym-Vitamin-Gemisch kann jedoch auch als gesonderter Wirkstoff mit einem Anteil von 0,01 bis 4 Gew-% in das erfindungsgemäße Kosmetikum eingebracht werden.

Für die Erfindung ist es vorteilhaft, wenn die Viskosität im Bereich von 100 bis 10.000, vorzugsweise 100 bis 3000 Pa·s liegt, gemessen mit einem Brookfield-Viskosimeter bei einer Temperatur von 25 °C und mit einer Spindel RV3-6 und T-B bis T-F. Dies betrifft jedoch nicht einen Puder, der die Schwammteilchen als Trockensubstanz enthalten kann.

Es ist weiterhin vorteilhaft, wenn das Kosmetikum wenigsten 30 Gew-% Wasser enthält, bezogen auf die Gesamtzusammensetzung, insbesondere 30-95 Gew-% Wasser und besonders bevorzugt 55-80 Gew-% Wasser.

Das erfindungsgemäße Kosmetikum kann weiterhin kosmetische Hilfs- und Trägerstoffe enthalten, wie sie üblicherweise in solchen Zubereitungen verwendet werden, z.B. Wasser, Konservierungsmittel, Farbstoffe, Pigmente mit färbender Wirkung, Verdickungsmittel, Duftstoffe, Alkohole, Polyole, Ester, Elektrolyte, Gelbildner, polare und unpolare öle, Polymere, Copolymere, Emulgatoren, Wachse, Stabilisatoren.

Zu den in dem erfindungsgemäßen Präparat enthaltenen kosmetischen Wirkstoffen gehören z. B. organische wasserlösliche Lichtschutzmittel, Radikalfänger, Vitamine, Enzyme, pflanzliche Wirkstoffe, Polymere, Melanin, Antioxidationsmittel, entzündungswidrige natürliche Wirkstoffe, mit Sauerstoff beladene asymmetrische lamellare Aggregate gemäß WO 94/00109; Kaolin sowie mit SiO₂ modifiziertes Kaolin gemäß WO94/17588.

Auch ein Gemisch einer wäßrigen Extraktion der Ananasfrucht in verkapselter Form und der Rückstand einer wäßrigen Extraktion von Joghurt (jeweils bei 10-30 °C) zusammen in einem Gel kann als zusätzlicher Wirkstoff eingesetzt werden. Ein solches Gemisch kann zusammen mit einem einwertigen Alkohol eingebracht werden.

Für den Fall des Einsatzes von Lichtschutzfiltern sind wasserlösliche UVB-Filter z.B. Sulfonsäurederivate von Benzophenon oder von 3-Benzylidencampher oder Salze wie das Na- oder K-Salz der 2-Phenylbenzimidazol-5-sulfonsäure. Besonders vorteilhaft ist deren Zusatz im Bereich von 0,1 bis 8 Gew-%.

Zu Antioxidationsmitteln gehören Vitamine wie Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z.B α-Carotin, β-Carotin; Harnsäure und Derivate davon; α-Hydroxysäuren wie Citronensäure, Milchsäure, Apfelsäure; Stilbene und deren Derivate; sowie Granatapfelextrakte.

Das erfindungsgemäße Kosmetikum kann als Emulsion oder Gel vorliegen.

Die für eine Emulsion eingesetzten öle können übliche kosmetische Öle sein, wie ein Mineralöl; hydriertes Polyisobuten); synthetisches oder aus Naturprodukten hergestelltes Squalan (INCI-Name: Squalane, z.B. Synthesqual®, Cosbiol®); kosmetische Ester oder Ether, die verzweigt oder unverzweigt, gesättigt oder ungesättigt sein können; pflanzliche öle; oder Gemische zweier oder mehrerer davon.

Besonders geeignete öle sind beispielsweise Mineralöle, Hydrogenated Polyisobuten, Polyisopren, Squalane, Tridecyltrimellitat, Trimethylpropan-triisostearat, Isodecylcitrat, Neopentylglycol-diheptanoat, PPG-15-stearylether sowie pflanzliche öle, wie Calendulaöl, Jojobaöl, Avocadoöl, Macadamianußöl, Rizinusöl, Weizenkeimöl, Traubenkernöl, Kukuinußöl, Distelöl, Nachtkerzenöl, Safloröl oder ein Gemisch mehrerer davon.

Zu geeigneten Gelbildnern für ein kosmetisches Gel gehören Carbomer, Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate, Polyvinylalkohol, Polyvinylpyrrolidon, Montmorillonit.

Als Hilfsstoffe verwendete Pigmente, Pigmentgemische oder Pulver mit pigmentartiger Wirkung, worunter auch solche mit Perlglanz-Effekt zu verstehen sind, können zum Beispiel umfassen Eisenoxide, natürliche Aluminiumsilicate wie Ocker, Titan(di)oxid, Glimmer, Kaolin, manganhaltige Tone wie Umbra und roter Bolus, Calciumcarbonat, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile, verkapselte und unverkapselte Getreidestärken sowie Glimmer-Titanoxid-organischer Farbstoff.

Eine besondere Wirkung der erfindungsgemäßen Kosmetikums besteht in seiner langanhaltenden feuchtigkeitsspendenden Wirkung.

Die Verwendung des erfindungsgemäßen Kosmetikums kann z.B. erfolgen in Form von Sonnencremes, Sonnengelen, After-sun-Produkten, Tagescremes, Nachtcremes, Masken, Körperlotionen, Reinigungsmilch, Körperpuder, Augenkosmetik, Haarmasken, Haarspülungen, Haarshampoos, Badeölen und in Produkten der dekorativen Kosmetik wie Deo-Stiften, Parfüm-Stiften, Lippenstiften, Gelen, Lidschatten, Kompaktprodukten wie Kompaktpuder oder Kompaktwachs, Rouge, Grundierung, Make-up usw. Die Herstellung derartiger Produkte erfolgt auf eine Weise, wie sie dem Fachmann auf diesem Gebiet bekannt ist.

Eine besonders hervorzuhebende zusätzliche Wirkung der Schwammteilchen in einem kosmetischen Puder besteht darin, daß ein solcher Puder als Mattierung wirkt.

Die Erfindung soll nachstehend durch Beispiele näher erläutert werden. Alle Angaben erfolgen in Gewichtsprozent, sofern nichts anderes angegeben ist.

### Beispiel 1 Gel für die Augen

| **Phase A** | |
|---|---|
| Wasser | q.s. ad 100 |
| Euspongia officinalis 30-60 µm | 0,01 |
| Carbomer | 0,2 |
| Glycerin | 5,0 |

| **Phase B** | |
|---|---|
| Triethanolamin | 0,2 |

| **Phase C** | |
|---|---|
| Apfelextrakt * | 1,5 |
| Parfüm | 0,5 |
| Konservierungmittel | 0,3 |

| | |
|---|---|
| * alkoholischer Extrakt aus roten Äpfeln | |

Die Verarbeitung erfolgt bei Raumtemperatur (etwa 22-25 °C). Zu Wasser werden die gewaschenen und entkalkten schwammteilchen (Eusp. off.), die auf eine Teilchengröße von 30-60 µm zerkleinert worden waren, gegeben und ca. 20 Min. bei etwa 300 U/min gerührt. Nach der Zugabe von Glycerin und Carbomer wird das Gemisch homogenisiert. Nach Zugabe von Phase B wird ebenfalls homogenisiert, und danach wird die Phase C unter Rühren zugegeben. Es bildet sich ein klares Gel mit leichter transparenter Struktur.

### Beispiel 2 Creme mit langandauernder Feuchtigkeitsabgabe

| **Phase A** | |
|---|---|
| Wasser | q.s. ad 100 |
| Glycerin | 5,0 |
| Propylene Glycol | 3,0 |

| **Phase B** | |
|---|---|
| Cetyl Alcohol | 2,0 |
| Petrolatum | 1,2 |
| Glyceryl Stearate | 2,0 |
| Steareth 21 | 3,0 |
| Shea Butter | 0,5 |

| **Phase C** | |
|---|---|
| Cyclomethicone | 5,0 |

| **Phase D** | |
|---|---|
| Euspongia officinalis 60-100 µm/Hyaluronic Acid * | 5,0 |
| Parfüm | 0,3 |
| Konservierungsmittel | 0,4 |

| | |
|---|---|
| * die Schwammteilchen wurden etwa 1 Stunde unter langsamem Rühren bei etwa 200-250 U/min mit Hyalyronsäure getränkt. | |

Die Phasen A und B wurden separat hergestellt unter Rühren bei etwa 70 °C. Sie wurden zusammengegeben und homogenisiert. Nach Abkühlung auf 50 °C wurde die Phase C hinzugegeben und das Gemisch homogenisiert. Danach wurde auf etwa 30 °C abgekühlt und die Phase D untergerührt für etwa 20 min.

### Beispiel 3

Es wurde wie im Beispiel 2 verfahren mit Ausnahme dessen, daß anstelle von Hyaluronsäure ein Hyaluronat-Wirkstoff-Wasser-Komplex gemäß Beispiel 2 der DE 199 23 829 A1 hinzugegeben wurde. Die langanhaltende Feuchtigkeitsabgabe wurde dadurch verstärkt, daß D-Panthenol, Sanddornöl und Nachtkerzenöl eine hautberuhigende und reizlindernde Wirkung ausübten, die insbesondere nach erhöhter Sonneneinstrahlung auf die Haut sehr überzeugend war.

## Patentansprüche

1. Kosmetikum mit erhöhter Feuchthaltewirkung, **dadurch gekennzeichnet, daß** es enthält 0,01 bis 5 Gew-%, bezogen auf das Gesamtgewicht des Kosmetikums, elastische Fasergerüstteilchen der Hornschwammarten Euspongia officinalis, Spongia usitatissima, Hippospongia equina und Gemische davon, in gereinigter und entkalkter Form, wobei die Teilchengrößen im Bereich von 10 bis 200 µm liegen, sowie 95 bis 99,99 Gew-% kosmetische Trägerstoffe, Hilfsstoffe, Wirkstoffe und Gemische davon.

2. Kosmetikum nach Anspruch 1, **dadurch gekennzeichnet, daß** die Teilchengrößen des Schwamms im Bereich von 30 bis 80 µm liegen.

3. Kosmetikum nach Anspruch 1, **dadurch gekennzeichnet, daß** die Fasergerüstteilchen des Schwammes verteilt in einem Gel vorliegen.

4. Kosmetikum nach Anspruch 3, **dadurch gekennzeichnet, daß** das Gel in der wäßrigen Phase einer O/W oder W/O-Emulsion enthalten ist.

5. Kosmetikum nach Anspruch 1, **dadurch gekennzeichnet, daß** die Fasergerüstteilchen des Schwammes verteilt in einem Puder vorliegen.

6. Kosmetikum nach Anspruch 1, **dadurch gekennzeichnet, daß** der Gehalt an Wasser 30 bis 95 Gew-% beträgt, vorzugsweise 55 bis 80 Gew-% beträgt bezogen auf das Gesamtgewicht des Kosmetikums.

7. Kosmetikum nach Anspruch 1, **dadurch gekennzeichnet, daß** die Viskosität in wäßriger Phase im Bereich von 100 bis 10.000 Pa·s liegt, gemessen nach der Brookfield-Methode.

8. Kosmetikum nach Anspruch 1, **dadurch gekennzeichnet, daß** die Fasergerüstteilchen des Schwammes im Gemisch mit feinteiliger Perlensubstanz, bestehend aus Aragonit oder Calcit oder Gemischen davon und organischen Bindemitteln (Conchagene), vorliegt.

9. Kosmetikum nach Anspruch 1, **dadurch gekennzeichnet, daß** die Fasergerüstteilchen des Schwammes im Gemisch mit Hyaluronsäure, Hyaluronsäurederivaten oder mit einem Hyaluronat-Wirkstoff-Wasser-Komplex vorliegen.

10. Kosmetikum nach Anspruch 1, **dadurch gekennzeichnet, daß** es zusätzlich wasserlösliche Lichtschutzmittel enthält.

## Claims

1. A cosmetic composition with an increased moisturizing effect comprising 0.01-5% by weight, relative to the total weight of the cosmetic composition, of elastic fibre structure particles of the horn sponge species Euspongia officinalis, Spongia usitatissima, Hippospongia equina and mixtures thereof in a cleaned, decalcified form and with particles sizes ranging between 10 and 200 µm, and 95-99.99% by weight of cosmetic carriers, auxiliaries, active agents and mixtures thereof.

2. A cosmetic composition according to Claim 1, wherein the particle size of said sponge ranges between 30 and 80 µm.

3. A cosmetic composition according to Claim 1, wherein said fibre structure particles of the sponge are distributed in a gel.

4. A cosmetic composition according to Claim 3, wherein said gel is contained in the aqueous phase of an O/W or W/O emulsion.

5. A cosmetic composition according to Claim 1, wherein said fibre structure particles of the sponge are distributed in a powder.

6. A cosmetic composition according to Claim 1, wherein water makes up 30-95% by weight, preferably 55-80% by weight, relative to the total weight of the cosmetic composition.

7. A cosmetic composition according to Claim 1, wherein the viscosity in an aqueous phase ranges between 100 and 10,000 Pa·s, measured according to the Brookfield method.

8. A cosmetic composition according to Claim 1, wherein said fibre structure particles of the sponge are mixed with comminuted pearl substance consisting of aragonite or calcite or mixtures thereof and organic binders (conchagens).

9. A cosmetic composition according to Claim 1, wherein said fibre structure particles of the sponge are mixed with hyaluronic acid, hyaluronic acid derivatives or with a hyaluronate-active agent-water complex.

10. A cosmetic composition according to Claim 1, wherein said composition in addition contains water-soluble sunscreens.

## Revendications

1. Composition cosmétique à effet hydratant augmenté, **caractérisée en ce qu'**il contient 0,01 à 5% en poids, sur base du poids total de la composition cosmétique, des parties squelettiques fibreuses élastiques des type d'éponge cornée Euspongia officinalis, Spongia usitatissima, Hippospongia equina et leurs mélanges, sous forme purifiée et décalcarisée, où la taille des particules se situe dans l'intervalle allant de 10 à 200 µm, ainsi que 95 à 99,99% en poids dé supports, auxiliaires et agents actifs cosmétiques, et leurs mélanges.

2. Composition cosmétique selon la revendication 1, **caractérisée en ce que** la taille des particules d'éponge se situe dans l'intervalle allant de 30 à 80 µm.

3. Composition cosmétique selon la revendication 1, **caractérisée en ce que** les particules squelettiques fibreuses d'éponge sont présentes sous forme distribuée dans un gel.

4. Composition cosmétique selon la revendication 3, **caractérisée en ce que** le gel est présent dans la phase aqueuse d'une émulsion H/E ou E/H.

5. Composition cosmétique selon la revendication 1, **caractérisée en ce que** les particules squelettiques fibreuses d'éponge sont présentes sous forme distribuée dans une poudre.

6. Composition cosmétique selon la revendication 1, **caractérisée en ce que** la teneur en eau se situe dans l'intervalle allant de 30 à 95% en poids, de préférence de 55 à 80% en poids, sur base du poids total de la composition cosmétique.

7. Composition cosmétique selon la revendication 1, **caractérisée en ce que** la viscosité de la phase aqueuse se situe dans l'intervalle allant de 100 à 10 000 Pa.s, mesurée par le procédé de Brookfield.

8. Composition cosmétique selon la revendication 1, **caractérisée en ce que** les particules squelettiques fibreuses d'éponge sont présentes en mélange avec des substances en perle, consistant en l'aragonite ou la calcite ou leurs mélanges, et des liants organiques (conchagène).

9. Composition cosmétique selon la revendication 1, **caractérisée en ce que** les particules squelettiques fibreuses d'éponge sont présentes en mélange avec l'acide hyaluronique, des dérivés de l'acide hyaluronique ou un complexe hyaluronate-agent actif-eau.

10. Composition cosmétique selon la revendication 1, **caractérisée en ce qu'**elle contient en outre, des agents de protection contre la lumière, solubles dans l'eau.
